# EUROPÄISCHE PATENTSCHRIFT

(11) **EP 1 997 429 B1**
(45) Veröffentlichungstag und Bekanntmachung des Hinweises auf die Patenterteilung: **23.11.2011**
(21) Anmeldenummer: 07010561.4
(22) Anmeldetag: 29.05.2007
(51) Int. Cl.: A61B 5/15

(54) **Flexible Lanzette in einem Lanzettensystem**
Flexible Lancet in a Lancet System
Lancet flexible en une systeme de lancettes

(43) Veröffentlichungstag der Anmeldung: 03.12.2008
(73) Patentinhaber: Roche Diagnostics GmbH, 68305 Mannheim (DE); F.Hoffmann-La Roche AG, 4070 Basel (CH)
(72) Erfinder: Kraemer, Uwe, 68549 Ilvesheim (DE); Hoenes, Joachim, 64673 Zwinenberg (DE)

(56) Entgegenhaltungen:
- WO-A-2005/117721
- DE-A1- 3 217 757
- US-A- 5 630 828

## Beschreibung

### Technisches Gebiet

Die Erfindung liegt auf dem Gebiet der Stechhilfen zur diagnostischen Ermittelung von Blutparametem.

### Stand der Technik

Die Gewinnung und Analyse von Körperflüssigkeiten findet auf vielen Gebieten der medizinischen Diagnostik statt. Deshalb ist es wünschenswert auch Routinetests außerhalb des Laboratoriums schnell und reproduzierbar zu ermöglichen. Das Testen kann mit verschiedenen Körperflüssigkeiten durchgeführt werden, wie z. B. Blut und/oder interstitieller Flüssigkeit. Diese Flüssigkeiten können auf verschiedene Charakteristiken hin untersucht werden. Die Ergebnisse dieser Untersuchung sind wichtig, um verlässliche Diagnosen, therapeutische Maßnahmen und Therapieverfolgungen durchführen zu können.

Die Analyse von Körperflüssigkeiten beginnt mit der Gewinnung der Flüssigkeit. Eine Methode zur Gewinnung von Körperflüssigkeit besteht darin, eine minimale Wunde in die Haut des Patienten mit Hilfe einer Nadel, Lanzette oder eines Messers zu erzeugen. Die dabei gewonnene Körperflüssigkeit kann entweder in kleinen Gefäßen gesammelt werden oder direkt in Kontakt mit einem Testelement wie z. B. einem Teststreifen zur Analyse gebracht werden. Um bei der Benutzung der Lanzetten, Nadel oder Klingen eine Verletzungsgefahr des Patienten zu vermeiden, wird die Stechhilfe mit einem Schutz an der Lanzettenspitze konstruiert. Die meisten dieser Stechhilfen benötigen ein manuelles Einfügen der Lanzette in die Stechhilfe. Dies ist bei einer sehr häufigen Benutzung der Stechhilfe eine sehr umständliche Handhabung. Eine Magazinierung von Lanzetten könnte dieses Problem beheben, wobei hier viele Sicherheitsaspekte zu beachten sind. Es ist beispielsweise zu beachten, dass die Sicherheit des Patienten bei der Benutzung der Stechhilfe gewährleistet ist. Zudem sollte das System nicht zu komplex werden, da es sonst vom Patienten nicht gut handhabbar wäre.

Im Stand der Technik werden hierzu einige Lösungen aufgezeigt. Das US Patent 2003/0199902 gewährleistet eine Versiegelung jeder einzelnen Lanzette in einem Magazin, wobei ein aufwendiger und Raum erfüllender Zahnradmechanismus dazu benutzt wird, die Lanzetten aus dem

Magazin zu befördern.

In der europäischen Anmeldung EP 1 203 563 wird ein analytisches Hilfsmittel beschrieben, das auf einem Trägerband ein Testelement aufweist, wobei auf diesem Testelement ein zusätzliches Rahmenelement aufgebracht ist, das beweglich ist und eine Lanzette beinhaltet. Das Rahmenelement kann bei Benutzung von einer parallelen Stellung zum Testelement in eine orthogonale Stellung bewegt werden, so dass die Lanzette durch eine Öffnung im Testelement aktuiert werden kann. Dies ist eine recht aufwendige Realisierung von einer Kombination von Testelement und Lanzette, da viele Teile mechanisch bewegt werden müssen und das System in seiner funktionsfähigen Form viel Platzbedarf hat.

Eine weitere europäische Anmeldung mit der Nummer EP 1 360 935, beschreibt eine Anordnung von Lanzetten (hier als "Tester" bezeichnet), um Zugang zu flüssigen Proben zu erhalten. Die Lanzetten sind dabei seriell auf einem Band angeordnet, das auf seiner Oberseite eine Abdeckung der Lanzetten aufweist. Um die Lanzette zur Benutzung freizulegen, wird auch hier ein aufwendiges mechanisches System benutzt, da der gesamte Lanzettenkörper zunächst aus der Bandebene heraus bewegt werden muss, um die Lanzette benutzen zu können.

In der US 20040193202 wird ein Lanzettenband beschrieben, bei dem ein Teil des Lanzettenkörpers gegenüber dem Band abknickbar ist. Bei dieser Anordnung von Band zu Lanzette ist der Nachteil, dass bei einer gewünschten Stechbewegung nicht nur die Lanzette bewegt werden kann, sondern immer Lanzette und Band zusammen bewegt werden muss. Dies führt dazu, dass ein Antriebsmechanismus gewählt werden muss, der auf Band inklusive Lanzette angepasst ist. Außerdem ist auch hier aufgrund dieser Einschränkung eine platzsparende bzw. unaufwendige Ausgestaltung einer einfachen und leichten Stechhilfe kaum möglich.

Dieser Stand der Technik weist diverse Nachteile auf. Es sind viele mechanische Schritte notwendig, um das einzelne Stechelement aus der Magazinierung, bei der die Lanzetten in einer seriellen Anordnung, also in der Trägerbandebene liegen, in eine Anordnung zu bewegen, bei der die Lanzette senkrecht zur Trägerbandebene angeordnet ist. Dies hat aufgrund der aufwendigen Mechanik zusätzlich den Nachteil, dass ein großer Platzbedarf für diese Mechanik benötigt wird. Ein weiterer Nachteil vieler Systeme aus dem Stand der Technik ist die aufwendige Entsiegelung der Lanzetten vor dem Stechvorgang.

Aus den Nachteilen des Standes der Technik ergibt sich folgende Aufgabe. Es soll eine Platz sparende, mit wenig mechanischem Aufwand benutzbare, magazinierbare Stechhilfe zur Verfügung gestellt werden, die eine einfache Handhabung möglich macht.

Diese Aufgabe wird durch den Gegenstand der Erfindung, wie er in den unabhängigen Patentansprüchen charakterisiert wird, gelöst. Bevorzugte Ausführungsformen sind Gegenstand der abhängigen Ansprüche.

Gegenstand der Erfindung ist ein System zur Gewinnung von Körperflüssigkeit mit einem Gehäuse mit einer Öffnung, die mindestens eine Lanzette beinhaltet, wobei die Lanzette in ihrer ursprünglichen Form länglich und gerade ist und ein distales Ende mit einer Lanzettenspitze (im Folgenden auch Spitze genannt) aufweist. Die ursprüngliche Form entspricht hierbei der Form der Lanzette bevor sie das erste Mal mit einem Führungselement in Kontakt tritt. Weiterhin beinhaltet das System eine Antriebseinheit sowie ein Führungselement, das mit der Lanzette mindestens während des Stechvorgangs in Kontakt tritt und dabei die Lanzette in ihrer Bewegung führt, so dass die Lanzette in Bezug auf die Antriebsrichtung gebogen wird.

Die Lanzette besitzt bevorzugterweise eine so große Rückstellkraft, dass die Lanzette ohne Kontaktierung mit dem Führungselement ihre ursprüngliche, gerade Form im wesentlichen annimmt.

Die Lanzette besitzt eine längliche Ausdehnung, mit einem proximalen und einem distalen Ende, wobei das distale Ende zum Zwecke des Einstechens in einen Körper besonders ausgeformt ist, beispielsweise in Form einer Spitze. Die Spitze ist dabei ein Punkt der sich am distalen Ende der Lanzette befindet, in den die Seitenflächen des länglichen Lanzettenkörpers zusammenlaufen. Die in der Spitze mündenden Seitenflächen der Lanzette können dabei zusätzlich geschärfte Kanten aufweisen. Die Lanzette besteht also mindestens aus einem Lanzettenkörper, der vorwiegend nahezu parallel verlaufende Seitenflächen- bzw. kanten aufweist und einem Bereich der Spitze oder Spitzenbereich, der sich direkt an den Lanzettenkörper anschließt und aufeinander zu laufende Seitenkanten aufweist, die in der Spitze münden. Der Bereich der Spitze, oder auch Spitzenbereich, kann also je nach Länge der auf einander zu laufenden Seitenkanten, unterschiedlich groß sein.

Die Lanzette ist bevorzugterweise eine Flachlanzette, die an ihrer flachen Seite abgebogen wird. Aber auch runde Lanzetten sind verwendbar.

Zum Abbiegen der Lanzettenspitze weist das System ein Führungselement auf. Dieses Führungselement dient dazu, unter möglichst geringer Krafteinwirkung ein Abbiegen mindestens der Lanzettenspitze um 5° bis 90° von der Antriebsrichtung zu ermöglichen. Das Führungselement kann dabei verschiedene Formen aufweisen. Es kann beispielsweise einstückig oder mehrstückig sein. Es gibt durch seine Ausgestaltung die Abbiegerichtung und die Ausprägung der Abbiegung vor, in dem es bevorzugterweise selber eine gebogene Form aufweist. Es besteht aus Materialien, die geeignet sind, während des Kontakts mit der Lanzette ihre Form zu bewahren, um die Lanzette ausreichend führen zu können. Dies können beispielsweise Metall, Keramik, Glas oder Kunststoffe sein. Das Führungselement kann dabei aus einem durchsichtigen Material gefertigt sein, so dass eine Detektion in verschiedenen Wellenlängenbereichen durch das Führungselement hindurch möglich ist.

Weiterhin kann es die Lanzette nur an einer Seite führen oder aber die Lanzette bei der Führung komplett umschließen. Das Führungselement kann eine geschlossene Struktur, z. B. in Form einer Röhre oder eine offene Struktur, in Form einer Rinne oder Schiene aufweisen. Das Führungselement ist mindestens beim Abbiegevorgang der Lanzette unbeweglich gegenüber der bewegten Lanzette ausgestaltet, in dem das Führungselement beispielsweise an dem Magazin oder an dem Gehäuse der Stechhilfe mindestens vorübergehend befestigt ist. Die Lanzette kann bei dem Abbiegevorgang in ihrer gesamten Länge gebogen werden oder aber nur in Teilbereichen der Lanzette, wie beispielsweise dem Spitzenbereich der Lanzette. Das Führungselement umgibt bevorzugterweise mindestens einen Teil der Lanzette bei dem Abbiegevorgang. Dabei treten bevorzugterweise die Lanzettenspitze sowie die geschärften Kanten nicht mit dem Führungselement direkt in Kontakt, um sie nicht untauglich für den Stechvorgang zu machen in dem sie an Schärfe durch den Kontakt verlieren. Der weitere Teil des Lanzettenkörpers, der sich an den Biegebereich anschließt und im proximalen oder hintere Ende der Lanzette mündet, kann eine vom Spitzenbereich verschiedene Geometrie aufweisen, wie beispielsweise eine Verbreiterung oder Verdickung des Lanzettenkörpers. Dieser breitere Teil des Lanzettenkörpers kann zusätzlich härter gearbeitet sein, um eine höhere Stabilität gegen Verformung aufzuweisen. Dies kann beispielsweise durch die Wahl anderer Materialien geschehen oder durch die Wahl der Menge bzw. Dicke der verwendeten Materialien. Ist die Lanzette auf einem Träger angebracht, so kann mindestens der hintere Lanzettenkörperteil, der vorzugsweise eine festere Struktur gegen Verformung aufweist mit dem Träger verbunden sein, um eine stabile Verbindung mit dem Träger herstellen zu können. Der hintere Lanzettenkörperteil bis zum proximalen Ende der Lanzette, kann bevorzugterweise zur Ankopplung einer Antriebseinheit benutzt werden. Hierfür kann der Lanzettenkörper unterschiedliche Ankopplungsstrukturen aufweisen, wie beispielsweise Nuten, Löcher, Kerben oder Ausstülpungen. Dabei findet der Antrieb vorzugsweise in Ausrichtung der Längsachse der Lanzette statt, wobei die abgebogene Lanzettenspitze linear in ein Körperteil eingestochen werden kann. Dabei ist die Öffnung des Gehäuses vorzugsweise parallel zur Antriebsrichtung angeordnet. Durch die Ausrichtung der abgebogenen Lanzettenspitze, die von der Längsachse des Lanzettenkörpers verschieden ist ergibt sich der Vorteil, dass andere geometrische Anordnungen realisiert werden können als das mit der rein axialen Antriebsrichtung für Lanzettenkörper und Lanzettenspitze möglich ist. Des Weiteren ergibt sich durch das bevorzugte Abbiegen der Lanzettenspitze, die aufgrund ihrer Zweckbestimmung (möglichst schmerzarm in ein Körperteil eingestochen zu werden), möglichst dünn und fein ausgestaltet ist, dass nur wenig Kraft hierzu notwendig ist bzw. wenig morphologische Veränderung an der Lanzette vorgenommen werden muss, um ein leichtes Abbiegen zu ermöglichen. Hierdurch wird sowohl eine einfache Abbiegung der Lanzette gewährleistet, ohne dass eine erhöhte Labilität der Lanzette bei einer möglichen Magazinierung auf einem Träger oder Trägerband eintritt. Der Lanzettenkörper, der zum Ankoppeln der Antriebseinheit dient, kann unabhängig der Ausgestaltung des Spitzenbereiches stabil ausgefertigt sein, um den Beanspruchungen bei der Ankopplung der Antriebseinheit und den Kräften bei der Stechbewegung zu genügen. Dabei ist es dennoch möglich die Lanzette in ihrer kompletten Länge durch das Führungselement hindurch zu führen.

Mindestens die Lanzettenspitze kann mit einem Sterilschutz versehen sein, der bevorzugterweise beim Abbiegen der Lanzette geöffnet wird.

In einer Ausführungsform befindet sich die Lanzette auf einem Träger. Der Träger kann beispielsweise zur einfachen Magazinierung von mehreren Lanzetten dienen. Außerdem kann der Träger auch eine Schutzfunktion gegenüber äußeren Einflüssen (wie beispielsweise Stößen oder sonstige Berührung) für die Lanzette übernehmen, wenn die Lanzette beispielsweise zu mindestens einem Teil vom Träger umgeben ist. Dies ist besonders bevorzugt, wenn der Träger eine Trägerband ist. In einer bevorzugten Ausführungsform sind der Lanzettenkörper und die Lanzettenspitze im ungebogenen Zustand auf dem Träger, vorzugsweise liegend, angebracht..

Als Träger kann alternativ eine kreisförmige Struktur dienen, an oder auf der die mindestens eine Lanzette befestigt ist. Bevorzugterweise ist der Träger scheibenförmig ausgestaltet. Es sind aber auch andere Trägerstrukturen, die beispielsweise eckige, kugel- oder bandförmige, ovale oder elliptische Formen aufweisen, denkbar.

Alternativ können die Lanzetten auch in einem Stapelmagazin angeordnet sein, wobei die Lanzetten übereinander angeordnet sind. Dabei kann das Magazin Teil des Systems sein und austauschbar mit dem System wechselwirken. Alternativ kann das System im ganzen disposibel sein, in dem die Lanzetten ohne weiteres Magazin gelagert sind. Hierbei wird das gesamte System nach Verbrauch der magazinierten Elemente verworfen. Neben den Lanzetten als magazinierte Elemente, können außerdem z. B. Testelemente Bestandteil des Magazins oder des Systems sein.

Egal wie die Magazinierung vorgenommen wird, kann in einer alternativen Ausführungsform für jede Lanzette ein Führungselement mit bevorratet werden, dass zusammen mit der Lanzette in eine Einstichposition in dem System bewegt werden kann. Dabei kann das Führungselement in dieser Einstichposition, die sich direkt unter der Öffnung des Gehäuses befindet mit dem Gehäuse so wechselwirken, dass es dort gegenüber dem Gehäuse fixiert wird und sich nicht mit der Lanzette beim Einstichvorgang bewegt sondern sie nur führt.

Die Lanzette und der Träger können in einem Stück ausgebildet sein, wobei die Lanzette aus dem Träger herausbiegbar oder herauslösbar ausgestaltet werden kann. Dies ist bevorzugt, wenn die gesamte Struktur aus Metall, besonders bevorzugt aus Stahl gefertigt wird. Aber auch andere Materialien, wie beispielsweise Keramik oder Polymerstrukturen wären für eine einstückige Ausbildung von Lanzette und Träger möglich.

In einer alternativen Ausführungsform beinhaltet die Lanzette eine Struktur, die zur Aufnahme von Körperflüssigkeit geeignet ist. Dies kann bevorzugterweise eine Kapillarstruktur sein, aber auch alle alternativen Strukturen, wie beispielsweise Lochstrukturen, Spalt- oder Rinnenstrukturen sind zur Aufnahme von Flüssigkeit geeignet. Dabei kann auch durch eine Prägung im abgebogenen Bereich, bevorzugt im Spitzenbereich, eine Struktur dazu ausgebildet sein, Flüssigkeit aufzunehmen. Diese Ausführungsform wird im Folgenden als Microsampler bezeichnet, da eine Probenaufnahme durch die Lanzette und nicht direkt durch ein Testelement erfolgt. Die Struktur zur Aufnahme von Körperflüssigkeit kann sich bevorzugterweise im Spitzenbereich befinden. Sie kann in einer alternativen Ausführungsform auch über den Spitzenbereich hinausgehen und sich über Teile des Lanzettenkörpers erstrecken. Dabei kann die Struktur zur Aufnahme der Körperflüssigkeit in einem Stück ausgebildet sein oder in mehrere Bereiche unterteilt sein. In einer bevorzugten Ausführungsform beginnt diese Struktur zur Aufnahme von Flüssigkeit im Spitzenbereich und erstreckt sich über annähernd die gleiche Ausdehnung wie im Spitzenbereich in den Lanzettenkörper hinein. Hierbei kann die Struktur zur Aufnahme von Körperflüssigkeit in den Knickbereich münden oder über den Knickbereich hinaus in den Lanzettenkörper hineinragen. Die im Microsampler gesammelte Körperflüssigkeit kann anschließend auf ein Testelement übertragen werden und durch ein Detektionssystem (z. B. optisch oder elektrochemisch) detektiert und durch ein Auswertesystem ausgewertet werden.

Des Weiteren kann mindestens ein Testelement in, auf oder neben diesem Träger oder einem weiteren Träger angeordnet sein. Das Testelement dient zur Aufnahme der gewonnen Körperflüssigkeit und der anschließenden Detektion eines Analyten aus der Körperflüssigkeit. Dabei kann das Testelement Reagenzien zur Reaktion mit dem Analyten enthalten. Das Testelement kann auf einem separaten Träger angebracht sein, oder auf dem Träger der Lanzette. Um eine Kontamination der Spitze mit Substanzen aus dem Testelement zu vermeiden ist das Testelement bevorzugterweise nicht direkt mit der Lanzette verbunden sondern separat von der Lanzette auf dem Träger angeordnet. Die Anordnung des mindestens einen Testelements auf einem separaten Träger verstärkt diesen Effekt der verringerten Kontaminationsgefahr. In einer bevorzugten Ausführungsform sind Testelement und Lanzette so zueinander angeordnet, dass sie nach dem Abbiegevorgang der Lanzette durch eine zweite Bewegung der Lanzette oder des Testelementes in Kontakt treten können. Dies ist besonders bevorzugt, wenn die Lanzette in Form eines Microsamplers ausgebildet ist. Dies kann durch eine Bewegung der Lanzette relativ zum Träger durchgeführt werden. Eine Möglichkeit die Lanzette mit dem Testelement in Kontakt zu bringen ist ein Abknicken der Lanzette in die Abbiegerichtung, so dass die Lanzettenspitze mehr als 90° in Bezug auf den Lanzettenkörper abgebogen oder auch abgeknickt ist. Das Testelement befindet sich in dieser Ausführungsform bevorzugterweise auf einem Teil des Lanzettenkörpers. Eine alternative Bewegung der Lanzette zum Kontaktieren der Lanzette mit einem Testelement, ist eine Auslenkbewegung der Lanzette oder des Trägers in die, zur ersten Biegebewegung, entgegen gesetzten Richtung. Bei dieser Bewegung kann die Lanzettenspitze in die Ebene des Lanzettenkörpers zurückgebogen werden. Alternativ zur Kontaktierung von Testelement und Lanzette kann die Körperflüssigkeit auch direkt vom Körperteil des Benutzers auf das Testelement übertragen werden.

Eine weitere Alternative das Testelement mit der Lanzette zu kontaktieren ist die Bewegung des Testelementes selber. Hierzu befindet sich das Testelement bevorzugterweise auf einem zweiten Träger oder in einem zweiten Magazin, wobei zumindest zum Teil der Träger der Lanzette oder des Testelementes relativ zueinander beweglich angeordnet sind.

In einer bevorzugten Ausführungsform ist der Träger als Trägerband ausgestaltet, auf dem bevorzugt eine Mehrzahl Lanzetten positioniert sind. In dieser Ausführungsform wird eine Vorrichtung zur Gewinnung von Körperflüssigkeiten beschrieben, die ein im Wesentlichen planares Trägerband mit einer Längsausrichtung und einer Querausrichtung besitzt, auf der mindestens eine Lanzette, beinhaltend einen Lanzettenkörper und eine Spitze angeordnet ist, wobei die Lanzette liegend auf dem Trägerband, angeordnet ist. Bevorzugterweise besitzt die Lanzette eine ausreichend hohe Rückstellkraft, so dass sie in den Bereichen in denen sie das Führungselement kontaktiert, nach dem Kontakt mit dem Führungselement ihre ursprüngliche gerade Form im Wesentlichen wieder annimmt. Im Wesentlichen bedeutet in diesem Zusammenhang, dass der Biegungsgrad der Lanzette vor und nach dem Kontakt mit dem Führungselement nicht mehr als 10° voneinander abweicht. Dabei hängt die Rückstellkraft und damit auch die Steifigkeit der Lanzette von verschiedenen Parametern ab. Zum einen ist sie bestimmt durch das Material und dessen Eigenschaften, so sind spröde Materialien eher nicht geeignet, um gebogen zu werden. Materialien die eine zu niedrige Steifigkeit besitzen, wie beispielsweise dünne Elastomere oder Plastikfolien sind Materialien, die ebenfalls weniger geeignet sind, da sie keine ausreichende Stabilität für den Einstichvorgang besitzen. Die Lanzette sollte also aus einem Material gefertigt sein, das eine ausreichende Formstabilität bietet, so dass die Lanzette beim Stechvorgang nicht verformt wird, aber gleichzeitig eine ausreichende Flexibilität besitzt, um zwischen 10° und 90° gegenüber ihrer ungebogenen Form gebogen zu werden. Dabei soll das Material nach der Krafteinwirkung durch das Führungselement wieder annähernd in seine ursprüngliche Form zurückfinden. Diese Rückstellkraft kann beispielsweise bei Metallen, aber auch bei bestimmten Polymeren und deren Mischungen gefunden werden.

Die Steifigkeit soll als Maß für den Widerstand des Materials gegen elastische Verformung verstanden werden. Bevorzugterweise besitzt der zu biegende Teil der Lanzette erniedrigte Steifigkeit gegenüber dem restlichen Lanzettenkörper, so dass die Lanzette unter Krafteinwirkung bevorzugt in diesem Bereich gebogen werden kann. Dabei wird mindestens die Spitze in Bezug auf den restlichen Lanzettenkörper in ihrer Ausrichtung verändert. Diese Änderung der Ausrichtung ist bevorzugterweise aus der Antriebsebene bzw. Lanzettenkörperebene heraus. Dabei kann der Lanzettenkörper mindestens zu einem Teil in der Ursprünglichen Ebene bzw. der Trägerbandebene verbleiben, wenn er beispielsweise daran fixiert ist. Die Kraft, die zur Veränderung der Ausrichtung der Lanzetten benötigt wird ohne sie dabei zu verformen, wird auch als Schwellenkraft bezeichnet. Diese Schwellenkraft soll so groß sein, dass sie die Veränderung der Ausrichtung der Lanzette bewirkt, ist dabei aber so dimensioniert, dass keine ungewollten bleibenden Verformungen an Lanzette, am Träger oder am Trägerband auftreten.

Die Übertragung der Kraft auf die Lanzette kann durch ein Führungselement stattfinden, das mit der Lanzette in Kontakt tritt. Bei Lanzetten auf oder an einem Träger oder Trägerband, kann während des Stechvorgangs zumindest ein Rest des Lanzettenkörpers am Trägerband verbleiben.

In einer bevorzugten Ausführungsform ist das Führungselement zweiteilig. Hierbei wird die Lanzettenspitze durch den Stößel geradlinig angetrieben, wobei die Lanzette bereits mit dem Führungselement in Kontakt steht oder beim Antriebsvorgang in Kontakt damit gebracht wird. Die Lanzette kann also bereits vor dem Stechvorgang in dem Führungselement gebogen vorliegen oder aber ungebogen. Das Führungselement kann sich dabei an verschiedenen Stellen im System befinden. Auf diese Weise kann die Lanzette an verschiedenen Stellen im System gebogen werden, was eine hohe Flexibilität der Anordnung von Antriebselement und Lanzette sowie anderen Komponenten zueinander bewirkt. Besonders bevorzugt für die Anordnung auf einem Träger oder Trägerband ist dabei eine Flachlanzette.

Das Material der Lanzette ist bevorzugterweise Metall, besonders bevorzugt ist Stahl. Die Lanzette kann aber auch aus anderen Materialien bestehen, die es sowohl ermöglichen, dass die Lanzette bei Krafteinwirkung biegbar ist und dabei genügend Steifigkeit besitzt, um bei Benutzung in die Haut einzudringen ohne ihre Form zu verändern. Außerdem sollte das Material der Gestalt sein, dass es an dem distalen Ende der Lanzette zu einer scharfen Spitze gearbeitet werden kann, da sonst beim Stich zu viel Schmerz generiert wird. Die Herstellung von Lanzetten im Allgemeinen ist im Stand der Technik hinreichend bekannt, wie beispielsweise in DE 19 604 156 oder EP 0 565 970.

Das Trägerband ist bevorzugterweise aus einer Plastikfolie gefertigt. Dies kann aber auch ein anderes flexibleres Material sein, wie z. B. in der Anmeldung US 20050245845 beschrieben. In einem integrierten System kann auf dem Trägerband zusätzlich mindestens ein Testelement angeordnet sein. Bevorzugt werden Lanzette und Testelement alternierend angeordnet. Die Lanzette kann sowohl diagonal, in Längsausrichtung wie auch in Querausrichtung auf dem Band angebracht sein. Eine mögliche Ausführungsform ist die Anordnung von Lanzette und Testelement in direkter Nachbarschaft. Auf diese Weise ist ein direkter Transfer von Flüssigkeit nach dem Stechvorgang auf das Testelement möglich, ohne dass das Band weiter bewegt werden muss.

Zur Aktuierung der Lanzette werden im Folgenden verschiedene Möglichkeiten beschrieben. Die Lanzette kann an ihrem proximalen Ende auf dem Träger oder Trägerband so fixiert sein, dass ein Teil der Lanzette in Relation zum oder mit dem Träger oder Trägerband bewegt werden kann, während das proximale Ende an mindestens einem Punkt mit dem Träger oder Trägerband verbunden bleibt. Eine weitere bevorzugte Befestigung der Lanzette, ist die Fixierung des Lanzettenkörpers an dem Träger oder Trägerband, wobei sich der Spitzenbereich vom Träger oder Trägerband löst. Die gesteuerte Bewegung der Lanzette kann durch Bewegen des Träger oder Trägerbandes oder durch Ergreifen der Lanzette mit einem Greifelement geschehen, wobei die Lanzette mit oder ohne den Träger oder das Trägerband aus der Ebene des Trägers oder des Trägerbandes herausbewegt wird. Diese Bewegung kann mittels eines Antriebselementes durchgeführt werden, das parallel zur Träger oder Trägerbandebene auf die Lanzette Kraft überträgt. Die Kraftübertragung findet durch ein Antriebselement statt, das z. B. ein Stößel sein kann oder ein Greifelement, welches die Lanzette an ihrem Lanzettenkörper greift und bewegt. Hierbei ist in einer bevorzugten Ausführungsform die Einstichtiefe des Blutentnahmegerätes frei wählbar. Zur Regulierung der Einstichtiefe wird die Bewegung der Lanzette durch ein variierbares Anschlagselement definiert, gegen das die Lanzette während des Einstichvorgangs anschlägt. Die Lanzette kann hierzu am Lanzettenkörper Anschlagswiderstände in Form von beispielsweise Verdickungen oder Flügeln besitzen, die mit den Anschlagselement wechselwirken können, um die Bewegung der Lanzette zu stoppen. In Abhängigkeit von der Position des Anschlagelementes wird auf diese Weise die Länge der Lanzettenspitze, die aus der Gehäuseöffnung austritt und somit die Einstichtiefe variiert. Das Anschlagselement kann alternativ in das Gehäuse oder in das Führungselement integriert werden.

Zum Antrieb der Lanzette können ballistische oder Kulissen geführte Mechanismen benutzt werden, die aus dem Stand der Technik bekannt sind, wie z. B. in DE 19 604 156, EP 0 565 970, US 5,318,584 oder US 4,924,879 beschrieben. Eine bevorzugte Ausführungsform für den Antrieb der Lanzette ist die freie Bewegung der Lanzette nach Kraftübertragung durch das Antriebselement, wie beispielsweise den Stößel. In dieser Ausführungsform wird ein Impuls von dem Antriebselement auf die Lanzette übertragen und die Lanzette bewegt sich durch mindestens einen Teil des Führungselementes in Richtung Gehäuseöffnung.

Zur hygienischen Verwendung des Systems wird die Lanzette mindestens im Spitzenbereich durch einen Sterilschutz geschützt. Bevorzugterweise ist die Lanzette über den ganzen Lanzettenkörper mit dieser Folie überdeckt. Die Folie kann sich dabei auch über einen Teil des Trägerbandes bzw. Trägers erstrecken und ist damit verbunden. Dieser Sterilschutz kann aus einer Polymerschicht bestehen, die nach der Verknüpfung der Lanzette mit dem Trägerband bzw. Träger aufgebracht wird. Der Sterilschutz kann bei der Bewegung der Lanzette durch das Führungselement zerstört oder abgestreift werden und legt einen Teil der Lanzette, aber mindestens den Spitzenbereich der Lanzette frei. Alternativ kann der Sterilschutz vor Benutzung der Lanzette entfernt werden. Bevorzugterweise wird der Sterilschutz hierbei im Ganzen entfernt.

Gegenstand der Erfindung ist ebenfalls eine Stechhilfe zur Entnahme von Körperflüssigkeit. Diese Stechhilfe besteht bevorzugterweise aus einem Gehäuse, mit mindestens einer Öffnung, und mindestens einer Lanzette. In der Stechhilfe bzw. der Vorrichtung können herkömmliche Lanzetten benutzt werden, bevorzugterweise Flachlanzetten, aber auch alle Lanzetten, bei denen die Biegekraft des Führungselementes ausreicht, um mindestens einen Teil der Lanzette aus der Ebene der Antriebsrichtung herauszubewegen. Dabei tritt mindestens ein Teil der Lanzette aus der Gehäuseöffnung aus und sticht in die Haut des Patienten. Das Führungselement biegt dabei die Lanzette wir bereits erwähnt um 5° bis 90° von der Antriebsebene heraus. An der Einstichstelle bildet sich ein Bluttropfen, der zur Analyse benutzt wird. Wenn sich ein Testelement in der Stechhilfe befindet, so kann dies mit dem Bluttropfen in Kontakt gebracht werden indem das Testelement unterhalb der Gehäuseöffnung transportiert wird. Der Bluttropfen kann auf das Testelement aufgebracht werden, ohne dass der Patient weitere Schritte einleiten muss. Alternativ kann sich das Testelement auch auf einem zweiten Träger befinden, wie bereits beschrieben.

Das Blut reagiert mit einem oder mehreren Reagenzien, die sich auf dem Testelement befinden, wie sie z. B. aus den Dokumenten EP-A 0 885 591, EP-B 0 535 480 und EP-B 0 477 322 bekannt sind. Das Testelement wird mittels eines Detektors analysiert.

Das Blut kann auf verschiedene Komponenten hin untersucht werden, wie es im Stand der Technik bekannt ist. Zum Beispiel kann die Analyse auf Blutbestandteile wie Hämatokrit, Glucose, Cholesterin, Koagulation, Eisen und andere gerichtet sein. Zur Analyse können unterschiedliche Methoden zur Anwendung kommen. So können beispielsweise elektrochemische Nachweisreaktionen benutzt werden, aber auch optische (z. B. Reflektion, Absorption, Fluoreszenz, Raman-Spektroskopie) oder magnetische Nachweisreaktionen. Typischerweise wird die Flüssigkeit mit einem Testsystem in Kontakt gebracht, wobei eine Reaktion zwischen einem Testelement und der Flüssigkeit stattfindet. So beruht die Detektion mittels eines optischen Testelements auf einer Farbreaktion zwischen Flüssigkeit und Nachweisreagenz. Beispiele für diese Reaktionen sind in den US Patenten 3,802,842; 4,061,468 und 4,490,465 beschrieben.

Bei der Benutzung des Gerätes führt das System verschiedene Schritte durch. Befindet sich die Lanzette bereits in Kontakt mit dem Führungselement, so werden die Lanzette mit dem Führungselement zusammen in eine Position gebracht, in der die Lanzette durch die Öffnung des Gehäuses von dem Antriebselement hindurch angetrieben werden kann. Dabei wird vorzugsweise der Sterilschutz von der Lanzette zerstört. Bei dem Aktuationsvorgang tritt die Lanzette zumindest zu einem Teil in die Haut des Patienten ein und danach wieder in das Gerät zurück. Dabei kann im Falle der Verwendung eines Microsamplers Blut an der Lanzette gesammelt werden. Wird ein Transportband verwendet, wird dieses weiter transportiert und auf eine Spule gewickelt. Dabei liegt die Lanzette bevorzugterweise wieder flach auf dem Trägerband. Dieser Remagazinierungsvorgang wird in der Patentanmeldung US 20050245845 beschrieben.

In einem integrierten System, in dem auch Testelemente auf dem Träger oder Trägerband, vorzugsweise alternierend mit den Lanzetten aufgebracht sind, wird das Testelement nach dem Stechvorgang bis zur Gehäuseöffnung transportiert, um den Bluttropfen zur Analyse aufzunehmen. Das Testelement kann bis zum Detektor transportiert und dort vermessen werden. Im Falle der Verwendung eines Microsamplers wird das gesammelte Blut auf ein benachbartes Testelement übertragen. Wie bereits erwähnt, kann sich das Testelement auf dem gleichen Träger befinden oder auf einem zweiten Träger. Die beiden Träger sind dabei vorzugsweise beweglich zueinander angeordnet.

Gegenstand der Erfindung ist weiterhin eine Lanzette, die so ausgestaltet ist, dass sie mit dem Führungselement wechselwirken kann, so dass sie während oder vor dem Stechvorgang gebogen wird. Dies ist bevorzugterweise eine Flachlanzette, die genügend Spannkraft bzw. Rückstellkraft aufweist, dass sie ihre ursprüngliche Form ohne Kontaktierung mit dem Führungselement annimmt.

Die Biegung der Lanzette vor oder während des Stechvorgangs bietet verschiedene Vorteile für ein System zur Gewinnung von Körperflüssigkeit. Ein Vorteil ist die Unabhängigkeit der Anordnung der Öffnung am Gehäuse in Relation zur Antriebsrichtung. Bei geradlinigem Antrieb der Lanzette, muss das Antriebselement immer in gleicher Ebene zur Einstichrichtung angeordnet sein. Dies führt zwangsmäßig zu einer lang gestreckten Stechhilfe. Mit der Möglichkeit die Lanzette zu biegen, kann die Öffnung am Gehäuse an anderer Stelle positioniert werden, so dass sie für den Patienten angenehmer zu erreichen ist. Dies kann weiterhin zu Folge haben, dass das Gerät angenehmer in der Hand liegt und leichter benutzbar ist.

Außerdem können weitere Elemente, wie beispielsweise Testelemente oder Detektoren sowie Auswerteeinheiten ohne großen Aufwand neben der Öffnung des Gehäuses anzuordnen sind, da die Lanzette nicht ständig den Weg zur Gehäuseöffnung versperrt. So ist es einfacher die Öffnung sowohl für Lanzette als auch für das Testelement zugänglich zu machen. Weiterhin kann eine Detektionseinheit direkt gegenüber der Öffnung angeordnet werden, da weder die Lanzette noch eine Antriebseinheit diese Öffnung blockieren.

### Kurzbeschreibung der Figuren

- Figur 1a:: Schematische Darstellung einer Lanzette in Wechselwirkung mit einem Führungselement
- Figur 1b:: Schematische Darstellung einer Lanzette vor dem Einstich
- Figur 1c:: Schematische Darstellung einer Lanzette während der Einstichbewegung
- Figur 2a:: Schematische Darstellung der Queranordnung von Lanzetten auf dem Trägerband.
- Figur 2b:: Schematische Darstellung der Lanzetten in Längsanordnung auf dem Trägerband.
- Figur 3a:: Schematische Darstellung des Trägerbandes mit alternierender Anordnung von Testfeldern und längs angeordneten Lanzetten.
- Figur 3b:: Schematische Darstellungen des Trägerbandes mit alternierender Anordnung von Testfeldern und quer angeordneten Lanzetten.
- Figur 4:: Schematische Darstellung eines integrierten Gerätes mit Gehäuse und allen wichtigen Komponenten.

### Beschreibung der Figuren

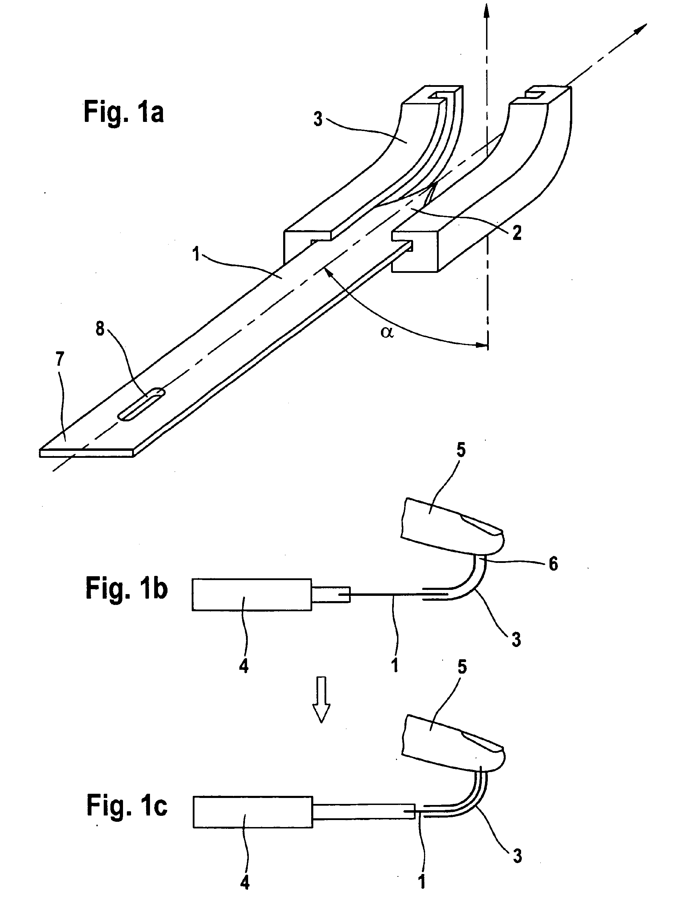

Figur 1a stellt eine mögliche Ausführungsform der Lanzette (1) dar. Die Lanzette (1) besitzt ein distales Ende (2) und ein proximales Ende (7). Sie steht in dieser Abbildung in Kontakt mit dem Führungselement (3), das sich entweder in einem Magazin für Lanzetten befindet oder in Verbindung mit dem Gehäuse der Stechhilfe steht, die hier nicht abgebildet ist. Das Führungselement (3) dient dazu, die Lanzette (1) vor oder während des Stechvorgangs um einen Winkel α, der zwischen 5° und 90° liegen kann, bevorzugterweise zwischen 40° und 90° liegt, aus der Antriebsebene heraus zubiegen. Zur Ankopplung einer Antriebseinheit (4) an die Lanzette (1) (wie in Fig. 1b und 1c gezeigt), besitzt die Lanzette (1) eine Ankopplungsvorrichtung, in diesem Fall in Form eines Lochs (8)

In Figur 1b wird die Lanzette (1) vor dem Stechvorgang gezeigt, wobei der Finger (5) des Patienten bereits am Gehäuse bzw. an dem Führungselement (3) anliegt. Die Lanzette (1) steht in Kontakt mit einem Antriebseinheit (4), das sie zum Stechvorgang bewegen kann. Dabei ist der Stechort die Öffnung (6) des Gehäuses, das hier nicht gezeigt ist. Die Öffnung (6) liegt um 90° gegenüber der Antriebsebene versetzt am Gerät vor. Um diesen Betrag wird die Lanzette vor oder während des Stechvorgangs gebogen. Dieser Biegungsgrad kann zwischen 5° und 90° liegen.

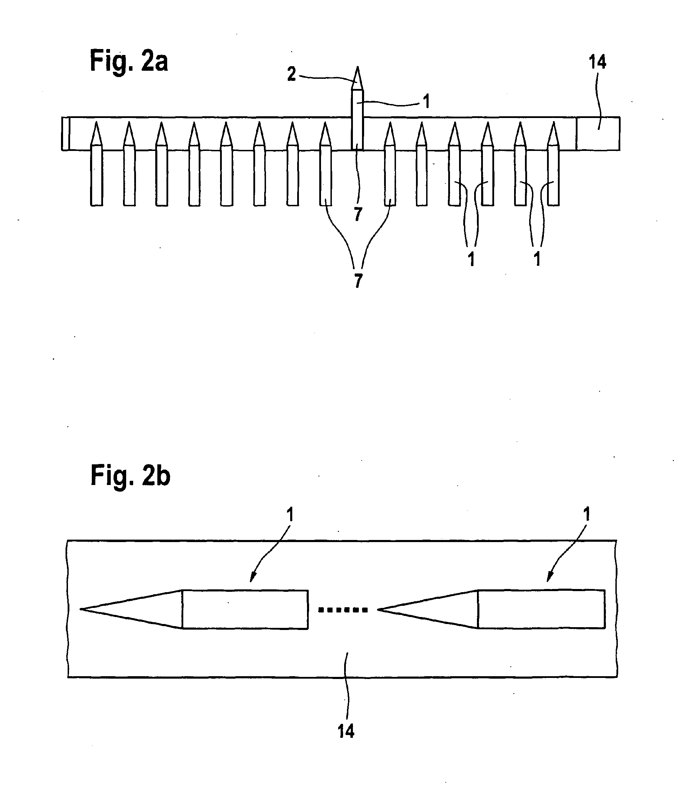

In Figur 2a ist ein Beispiel für eine Magazinierungsform der Lanzetten (1) auf einem Band (14) gezeigt. Es zeigt die Lanzetten (1) nach ihrer Herstellung. In dieser speziellen Ausführung sind die Lanzetten (1) durch Stanzen oder Ätzen aus einem dünnen Blechband, das hier das Trägerband (14) darstellt, herausgearbeitet. Die Lanzette (1) kann an einer Stelle mit dem Trägerband (14) weiter verbunden sein, dies ist bevorzugt am proximalen Ende (7) der Lanzette (1) möglich. Dabei ist die Lanzette (1) an dem Träger so lose angebunden, dass die Lanzette (1) mit einem wesentlichen Teil ihrer Länge mit einem hier nicht gezeigten Führungselement wechselwirken kann.

Hierbei kann die Verbindung der Lanzette durch ein Verbindungselement gewährleistet werden, so dass das Trägerband während des Antriebvorgangs nicht mitbewegt wird, die Lanzette aber in ihrer Bewegung ungestört ist. Dieses Verbindungselement bewirkt, dass die Lanzette nach dem Antriebs- bzw. Stechvorgang wieder auf dem Trägerband positioniert werden kann ohne dabei in Wechselwirkung mit anderen Lanzetten zu treten und sie zu kontaminieren. Dieses Verbindungselement kann beispielsweise aus einem elastischen Material wie Elastomeren oder sonstigen Kunststoffen bestehen.

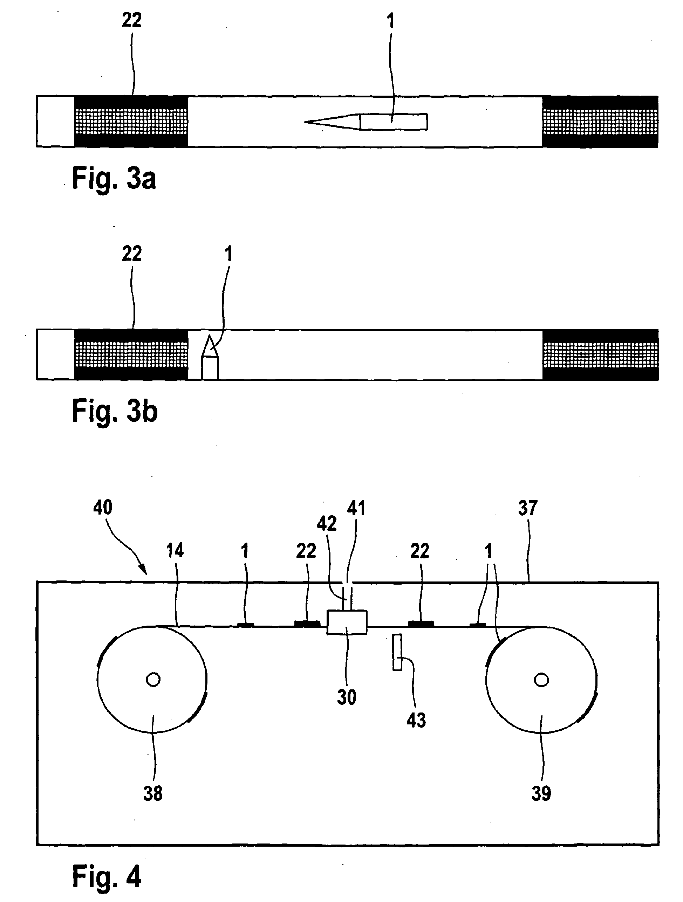

In Figur 3a wird ein Trägerband (14) gezeigt, auf dem alternierend ein Testfeld (22) und eine Lanzette (1) angeordnet sind. Dabei ist die Lanzette (1) in Längsausrichtung zum Trägerband angebracht. Der Abstand zwischen dem Testfeld (22) und der Lanzette (1) auf dem Trägerband (14) kann variieren. So ist es möglich, die Lanzette (1) so dicht neben dem Testfeld (22) zu platzieren, dass nach dem erfolgten Einstich die Flüssigkeit sofort von dem Testfeld (22) aufgenommen werden kann, ohne das Trägerband (14) zu bewegen. Eine weitere Ausführungsform mit alternierenden Testfeldern (22) und Lanzetten (1) ist in Abbildung 3b gezeigt. Hierbei ist die Lanzette (1) quer auf dem Trägerband angeordnet. Auch hier kann die Lanzette (1) in variablem Abstand zum Testfeld (22) platziert werden.

In Figur 4 ist ein integriertes System gezeigt. Das System besteht aus einem Gerät (40), das bevorzugterweise ein Gehäuse (37) mit einer Öffnung (41) sowie ein Trägerband (14), auf dem die Lanzetten (1) befestigt sind, besitzt. Das Trägerband (14) ist auf 2 Spulen (38) und (39) aufgewickelt. Dabei befindet sich der unbenutzte Teil der auf dem Trägerband befestigten Lanzetten auf der Spule (38) und der benutzte Teil wird auf die Spule (39) gewickelt. Zwischen den Spulen (38, 39) ist das Trägerband (14) gestreckt. Die Spulen (38, 39) werden durch einen Antrieb bewegt wie er aus dem Stand der Technik bekannt ist. Bevorzugterweise wird nur eine der beiden Spulen (38, 39) angetrieben. Ein Beispiel für einen solchen Antrieb ist in der Anmeldung US 20050245845 beschrieben. Die Lanzetten (1) befinden sich im ungebogenen Zustand auf dem Trägerband (14), wenn das Trägerband (14) auf die Spulen gewickelt ist. Zwischen den beiden Spulen (38, 39) befindet sich ein Führungselement (42), das die Lanzette (1) bei ihrem Antrieb durch das Antriebselement (30) in ihrer Bewegung führt und dabei zwischen 5° und 90 ° von der Antriebsrichtung abbiegt. In einer bevorzugten Ausführungsform des Führungselements (42), weist das Führungselement (42) eine zweiteilige Form auf, es kann jedoch auch in einem Stück ausgefertigt sein. Es kann mit dem Gehäuse (37) direkt verbunden sein, oder Bestandteil der Antriebseinheit (30) sein. Dabei kann sowohl die Antriebseinheit (30) als auch das Führungselement (42) im Gerät verschiebbar sein, so dass ein Detektor (43) oder andere Elemente des Systems unter die Öffnung (41) geschoben werde können.

Das Führungselement (42) kann ein Regelelement beinhalten (hier nicht gezeigt), das die Position des Führungselementes (42) so verändern kann, dass die Lanzette (1) unterschiedlich weit aus der Öffnung (41) aus dem Gehäuse (37) heraustreten lassen kann.

Zum Auslösen des Stechvorgangs wird das Trägerband (14) soweit transportiert, bis eine unbenutzte Lanzette (1) zwischen Gehäuseöffnung (41) und Stößel (30) liegt. Beim Auslösen des Stechvorgangs wird der Stößel (30) mit so viel Kraft auf die Lanzette (1) zu bewegt, dass mindestens die Lanzettenspitze (2) aus der Gehäuseöffnung (41) heraus bewegt wird. Nach erfolgtem Einstich wird das Blut auf einem Testfeld (22) gesammelt. Dort findet eine Reaktion des Blutes mit den Reagenzien auf dem Testfeld statt, die mit Hilfe eines Detektors (43) analysiert werden kann. Die Lanzette (1) wird zusammen mit dem Trägerband (14) remagaziniert. Durch ihre Rückstellkraft wird die Lanzette (1) wieder flach in das Trägerband (14) integriert.

## Patentansprüche

1. Eine System zur Entnahme von Körperflüssigkeit, beinhaltend:
- ein Gehäuse mit mindestens einer Öffnung (6),
- mindestens eine Lanzette (1), die in ihrer ursprünglichen Form länglich und gerade ist und ein distales Ende (2) mit einer Lanzettenspitze aufweist, um einen Stechvorgang vornehmen zu können,
- eine Antriebseinheit (4) zum Antreiben der Lanzette (1) in eine Antriebsrichtung, wobei das System mindestens ein Führungselement (3) aufweist, das mit der Lanzette (1) mindestens während des Stechvorgangs in Kontakt tritt und dabei die Lanzette (1) in ihrer Bewegung führt, so dass die Lanzette (1) in Bezug auf die Antriebsrichtung gebogen wird **dadurch gekennzeichnet dass** die Lanzetten (1) in einem Magazin untergebracht sind.

2. System nach Anspruch 1, **dadurch gekennzeichnet, dass** die Lanzette (1) eine Rückstellkraft besitzt, die ausreicht, damit die Lanzette (1) ohne Kontaktierung mit dem Führungselement (3) ihre ursprüngliche Form annimmt.

3. System nach Anspruch 1 oder 2, **dadurch gekennzeichnet, dass** die Lanzette (1) eine flexible Flachlanzette ist.

4. System nach Anspruch. 1, 2 oder 3, **dadurch gekennzeichnet, dass** die Öffnung (6) parallel zur Antriebsrichtung angeordnet ist.

5. System nach einem der vorhergehenden Ansprüche, **dadurch gekennzeichnet, dass** die Lanzette (1) während des Stechvorgangs durch das Führungselement (3) hindurch bewegt wird.

6. System nach einem der vorhergehenden Ansprüche, **dadurch gekennzeichnet, dass** das Führungselement (3) eine Schiene ist.

7. System nach einem der vorhergehenden Ansprüche, **dadurch gekennzeichnet, dass** die Lanzette (1) um 90° aus der Antriebsrichtung gebogen wird.

8. System nach einem der vorhergehenden Ansprüche, **dadurch gekennzeichnet, dass** das Führungselement (3) an dem Gehäuse fixiert ist.

9. System nach einem der vorhergehenden Ansprüche, **dadurch gekennzeichnet, dass** das Magazin das mindestens eine Führungselement (3) enthält.

10. System nach einem der vorhergehenden Ansprüche, **dadurch gekennzeichnet, dass** die mindestens eine Lanzette (1) auf einem Band (14) angeordnet ist.

11. Lanzette (1) zur Entnahme von Körperflüssigkeit geeignet zum Einsatz in einem System nach Anspruch 1 bis 10, die Lanzette (1) dazu ausgestaltet ist, mit dem Führungselewobei die Lanzette (1) dazu ausgestaltet ist, mit dem Führungselement (3) so Wechsel zu wirken, dass die Lanzette (1) in Bezug auf die Antriebsrichtung gebogen wird und die Lanzette (1) nach dem Stechvorgang aufgrund ihrer Rückstellkraft ihre ursprüngliche Form wieder annimmt.

12. Magazin zur Bevorratung von mehreren Lanzetten (1), beinhaltend:
- mehrere Lanzetten (1), die an ihrem distalen Ende (2) eine Spitze aufweisen,
- mindestens ein Führungselement (3),
**dadurch gekennzeichnet, dass** die Lanzette (1) mit dem Führungselement (3) beim Stechvorgang in Kontakt tritt und das Führungselement (3) die Lanzette (1) in ihrer Bewegung führt, so dass die Lanzette (1) in Bezug auf die Antriebsrichtung gebogen wird.

13. Magazin nach Anspruch 12, **dadurch gekennzeichnet, dass** das Magazin Lanzetten (1) bevorratet, die eine Rückstellkraft besitzen, die ausreicht, damit die Lanzette (1) ohne Kontaktierung mit dem Führungselement (3) ihre ursprüngliche Form annimmt.

## Claims

1. A system for withdrawing body fluid comprising:
- a housing with at least one opening (6)
- at least one lancet (1) which in its original form is elongate and straight and has a distal end (2) with a lancet tip in order to carry out a lancing process,
- a drive unit (4) for driving the lancet (1) in a drive direction, wherein the system has at least one guide element (3) which comes into contact with the lancet (1) at least during the lancing process and in doing so guides the movement of the lancet (1) such that the lancet (1) is bent relative to the drive direction, **characterized in that** the lancets (1) are accommodated in a magazine.

2. System according to claim 1, **characterized in that** the lancet (1) has a restoring force which is sufficient to enable the lancet (1) to adopt its original shape without contact with the guide element (3).

3. System according to claim 1 or 2, **characterized in that** the lancet (1) is a flexible flat lancet.

4. System according to claim 1, 2 or 3, **characterized in that** the opening (6) is arranged parallel to the drive direction.

5. System according to one of the previous claims, **characterized in that** the lancet (1) is moved through the guide element (3) during the lancing process.

6. System according to one of the previous claims, **characterized in that** the guide element (3) is a rail.

7. System according to one of the previous claims, **characterized in that** the lancet (1) is bent by 90° from the drive direction.

8. System according to one of the previous claims, **characterized in that** the guide element (3) is attached to the housing.

9. System according to one of the previous claims, **characterized in that** the magazine contains the at least one guide element (3).

10. System according to one of the previous claims, **characterized in that** the at least one lancet (1) is arranged on a tape (14).

11. Lancet (1) for withdrawing body fluid suitable for use in a system according to claim 1 to 10,
wherein the lancet (1) is designed to interact with the guide element (3) in such a manner that the lancet (1) is bent relative to the drive direction and the lancet (1) adopts its original shape again after the lancing process due to its restoring force.

12. Magazine for storing several lancets (1) comprising:
- several lancets (1) which have a tip at their distal end (2),
- at least one guide element (3),
**characterized in that** the lancet (1) comes into contact with the guide element (3) during the lancing process and the guide element (3) guides the movement of the lancet (1) such that the lancet (1) is bent relative to the drive direction.

13. Magazine according to claim 12, **characterized in that** the magazine stores lancets (1) which have a restoring force which is sufficient to enable the lancet (1) to adopt its original shape without contact with the guide element (3).

## Revendications

1. Un système de prélèvement de liquide organique, comprenant :
- un boîtier pourvu d'au moins une ouverture (6),
- au moins une lancette (1) dont la forme d'origine est allongée et droite et qui présente une extrémité distale (2) munie d'une pointe de lancette permettant d'effectuer une piqûre,
- une unité d'entraînement (4) permettant d'entraîner la lancette (1) dans une direction d'entraînement,
ledit système comportant au moins un élément de guidage (3) qui entre en contact avec la lancette (1) au moins pendant la piqûre et qui guide le mouvement de la lancette (1) de façon à recourber la lancette (1) par rapport à la direction d'entraînement, **caractérisé en ce que** les lancettes (1) sont logées dans un magasin.

2. Système selon la revendication 1, **caractérisé en ce que** la lancette (1) possède une force de rappel suffisante pour que la lancette (1) reprenne sa forme d'origine sans entrer en contact avec l'élément de guidage (3).

3. Système selon la revendication 1 ou 2, **caractérisé en ce que** la lancette (1) est une lancette plate flexible.

4. Système selon la revendication 1, 2 ou 3, **caractérisé en ce que** l'ouverture (6) est disposée parallèlement à la direction d'entraînement.

5. Système selon l'une des revendications précédentes, **caractérisé en ce que** la lancette (1) se déplace à travers l'élément de guidage (3) pendant la piqûre.

6. Système selon l'une des revendications précédentes, **caractérisé en ce que** l'élément de guidage (3) est un rail.

7. Système selon l'une des revendications précédentes, **caractérisé en ce que** la lancette (1) est recourbée de 90° par rapport à la direction d'entraînement.

8. Système selon l'une des revendications précédentes, **caractérisé en ce que** l'élément de guidage (3) est fixé sur le boîtier.

9. Système selon l'une des revendications précédentes, **caractérisé en ce que** le magasin contient ledit au moins un élément de guidage (3).

10. Système selon l'une des revendications précédentes, **caractérisé en ce que** ladite au moins une lancette (1) est disposée sur une bande (14).

11. Lancette (1) pour prélever du liquide organique apte à être utilisée dans un système selon la revendication 1 à 10,
ladite lancette étant conçue pour interagir avec l'élément de guidage (3) de façon que la lancette (1) soit recourbée par rapport à la direction d'entraînement et que la lancette (1) reprenne sa forme d'origine après la piqûre, grâce à sa force de rappel.

12. Magasin pour approvisionner plusieurs lancettes (1), comprenant :
- plusieurs lancettes (1) munies d'une pointe à leur extrémité distale (2),
- au moins un élément de guidage (3),
**caractérisé en ce que** la lancette (1) entre en contact avec l'élément de guidage (3) pendant la piqûre et l'élément de guidage (3) guide le mouvement de la lancette (1) de façon que la lancette (1) soit recourbée par rapport à la direction d'entraînement.

13. Magasin selon la revendication 12, **caractérisé en ce que** le magasin approvisionne des lancettes (1) possédant une force de rappel suffisante pour que la lancette (1) reprenne sa forme d'origine sans entrer en contact avec l'élément de guidage (3).
